Europäisches Patentamt

European Patent Office

Office européen des brevets

(19)

(11) Publication number: **0 111 696**
**B1**

# EUROPEAN PATENT SPECIFICATION

(45) Date of publication of patent specification: **07.01.88**

(51) Int. Cl.⁴: **A 61 M 1/34**

(21) Application number: **83110796.6**

(22) Date of filing: **28.10.83**

(54) **An arrangement for the removal of one or more fractions out of whole blood, plasma or other similar body fluids.**

(30) Priority: **10.12.82 SE 8207078**

(43) Date of publication of application:
**27.06.84 Bulletin 84/26**

(45) Publication of the grant of the patent:
**07.01.88 Bulletin 88/01**

(84) Designated Contracting States:
**AT BE CH DE FR GB IT LI LU NL**

(56) References cited:
**EP-A-0 046 470**
**WO-A-82/03331**
**US-A-4 043 501**
**US-A-4 127 111**

**BLOOD, vol. 58, no. 1, July 1981, pages 38-44, I.M. NILSSON et al.: "A procedure for removing high titer antibodies by extracorporeal protein-A-sepharose adsorption in hemophilia: Substitution therpy and surgery in a patient with hemophilia B and antiobodies" BLOOD**

(73) Proprietor: **Gambro Lundia AB**
**Box 10101**
**S-220 10 Lund (SE)**

(72) Inventor: **Larsson, Lars-Ake Lennart**
**Odlarevägen 66**
**S-240 21 Löddeköpinge (SE)**
Inventor: **Nilsson, Ake Wallys**
**Inspektorsgatan 8**
**S-244 00 Kävlinge (SE)**
Inventor: **Johansson, Bo Ingemar Willy**
**Lokföraregatan 3 D**
**S-222 37 Lund (SE)**
Inventor: **Nauclér, Lars Olof Vilhelm**
**Olshögsvägen 6**
**S-223 60 Lund (SE)**

(74) Representative: **Boberg, Nils Gunnar Erik**
**Gambro AB Patent Department Box 10101**
**S-220 10 Lund (SE)**

Courier Press, Leamington Spa, England.

## Description

### Technical Field

The present invention relates to an arrangement for the removal of one or more fractions out of whole blood, plasma or other similar body fluids with the help of adsorption on an adsorption column comprising means for feeding said body fluid and a regenerating fluid in turn to the adsorption column, and means for the removal of said fluids from the adsorption column, the means for the removal of the fluids comprising a valve means which is adapted to control the flow from the column in dependence of an analysis carried out by a first analysing device to feed said flow either to a second analysing device for a second analysis of the fluid or to the source of the body fluid, e.g. a patient, the fluid analysed by the second analysing device being fed either to a drain or to a collecting receiver.

Many diseases are due to the immunoprotection of the body having become unbalanced and undesirable fractions having been formed in the blood. For example in cases of haemophilia antibodies may be formed against factor VIII or factor IX which normally control the coagulation of the blood.

It is an object of the present invention to remove such undesirable fractions. It is another object to separate the removed fraction for further usage. For example the antibodies against the said factor VIII or factor IX in turn may be attached to a column for the separation of just factor VIII or just factor IX from other fluids such as blood or plasma from blood donors.

If it is desired to remove just the said antibodies against factor VIII or factor IX a column is used in accordance with the invention comprising just a factor VIII or factor IX attached to a carrier. Alternatively, for example, protein A can be bound to the carrier which in turn is capable of binding, among other things, several different types of IgG. It must be evident to those versed in the art that the invention can be used in conjunction with a number of different adsorption substances.

### Background Art

The invention can be said to constitute a direct further development of the technique which is described in the journal "Blood", Vol. 58, No. 1, July 1981 by Inga Marie Nilsson, Svante Jonsson, Siv-Britt Sundqvist, Åke Ahlberg and Sven-Erik Bergentz under the title "A Procedure for Removing High-Titre Antibodies by Extracorporeal Protein-A-Sepharose Adsorption in Haemophilia: Substitution Therapy and Surgery in Patients with Haemophilia B and Antibodies". It can also be applied, however, as mentioned previously, in a more general manner.

In accordance with the aforementioned paper it is known that, for example, plasma can be separated from whole blood and that subsequently factor VIII or factor IX antibodies can be separated from this plasma in one or the other of two columns connected in parallel containing protein A attached to agarose of the type which is sold under the name of Sepharose by Pharmacia Chemicals, Uppsala. When one column has become saturated the plasma in turn is passed over to the parallel column so as to make possible a regeneration of the firstmentioned column. Prior to this regeneration the remaining plasma in the column is pressed back to the patient with the help of a flushing liquid. Towards the end of the flushing the patient is disconnected and the flushing liquid is passed to a drain. Subsequently the pH of the column is lowed gradually by means of a further flushing through, this time with the help of a mixture of an acid and a base. When the attached fractions have become detached from the column, this is indicated on a succeeding UV-meter which in combination with a succeeding pH-meter conveys the flushing liquid together with separated fractions either to a drain or to a collecting receiver just for these fractions.

It was a disadvantage of this known system that it required a UV-meter of such sensitivity that it was unsuitable for disposable usage but had to be reused after sterilization. A further disadvantage was that the gradual lowering of the pH of the column took a relatively long time compared to the time required for the detachment of the collected fractions.

### Disclosure of Invention

In the arrangement in accordance with the invention a very simple analysing device for the coarser analysis of the fluid coming from the column is arranged instead. The fluid can be made for example to pass through a transparent glass pipe or plastic hose and its turbidity can be measured with the help of means known in themselves which do not come into contact with the fluid and consequently do not have to be sterilized. A possible finer analysis will take place instead after the point from where the fluid either is conducted to the patient or to the said drain or to the said collecting receiver for the separated fractions.

More particularly the invention relates to an arrangement of the abovementioned type characterized in that the first analysing device is for a coarser analysis of the fluids than the second analysing device.

Preferably the said first analysing device is constituted by a detector for the monitoring of the degree of dilution of the treated fluid, e.g. a whole blood detector in the treatment of blood or a plasma detector in the treatment of plasma. Detectors of this type may be of a very simple design and consequently be included appropriately in the total system as a disposable component.

Columns of the type described above can be very costly and are appropriately constructed, therefore, on a smaller scale. Because of this the arrangement in accordance with the invention is preferably provided with a valve device arranged upstreams of the column which is adapted so that it can convey alternately the treated fluid or a regenerating liquid respectively and/or a flushing liquid to the column.

The said regenerating liquid can be adapted so that in a known manner it gradually lowers the pH of the column during a certain period. Means are provided preferably to ensure that at the start of the period a rapid lowering is achieved so as to accelerate the starting of the regeneration, whereupon the lowering is adapted so as to occur slowly during the actual regeneration and preferably to be terminated rapidly during the finish of the said period.

The said second analysing device may be constituted of a pH meter. Alternatively it may be constituted by a protein detector for the monitoring of the separated fraction. However it is constituted appropriately by a device of each type.

If it is desired to collect the separated fraction it is appropriate to arrange downstream of the second analysing device a further valve device controlled by the same for the conveying of the separated fraction to the collecting receiver.

If the second analysis device is constituted by a pH meter as well as a protein detector it may be adapted so as to separate for collection all the fluid passed through which contains more than, for example, 20% protein and which has a pH value between for example 2 and 6.

Because the columns used, as has been mentioned, may be very expensive two or more substantially smaller such columns may be arranged in parallel connection for alternate adsorption and regeneration.

By way of examples of the contents of the columns it may be mentioned that experiments have been carried out with factor IX attached to agarose for the removal of antibodies against factor IX. In other experiments protein A has been used for the removal of one or more protein fractions of the IgG type. Protein A can be attached to larger or smaller agarose balls. Larger balls are used in the treatment of whole blood whilst the smaller balls can be used for the treatment of pure plasma.

Brief Description of Drawings

The invention will be described in more detail in the following with reference to the attached drawings which show a preferred embodiment of the subject of the invention and illustrates in diagrammatic form how the arrangement can be utilized.

Fig. 1 shows schematically an arrangement in accordance with the invention intended in particular for the treatment of plasma. By excluding the plasma filter present it can also be used, however, for example, for the direct treatment of whole blood.

Fig. 2 shows how the pH of the fluid treated is appropriately controlled at the input side of the column.

Fig. 3 shows in the same manner but with a certain shift in time in relation to fig. 2 how the pH and protein content of the fluid vary at the output end of the column.

Fig. 4 finally shows during which periods plasma can be returned to the patient or when the fluid treated is conducted to a drain or to a collecting receiver for the required separated fraction.

Best Mode of Carrying Out the Invention

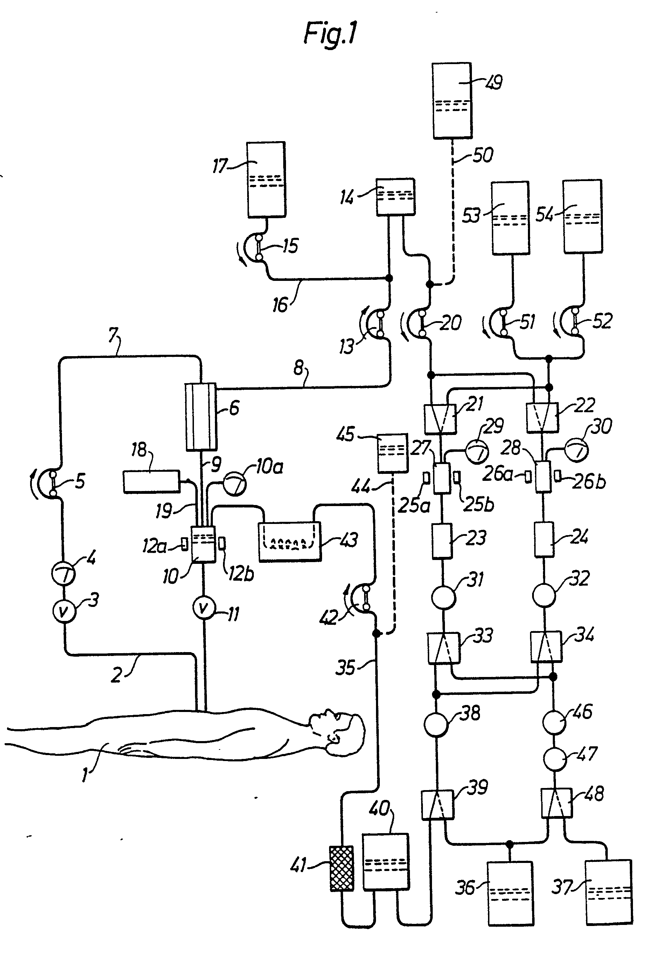

In fig. 1 numeral 1 designates the source of the fluid treated. This source, for example, may be a patient.

From the source 1 the fluid is passed through a line 2, e.g. a plastic tube provided with cannula via a shut-off clip 3, an arterial pressure gauge 4 and a blood pump 5 to a plasma filter 6. At a point 7 heparin is added so as to prevent coagulation of the blood. This heparin addition may be done possibly already in the canulla introduced into the patient.

Through a line 8 plasma is withdrawn for further treatment. Red blood-corpuscles and similar molecules or cells which do not pass the membrane in the filter 6 are conducted instead together with a smaller portion of plasma through a line 9 via a drip chamber 10 and a further shut-off clip 11 back to the patient. The drip chamber 10 is suitably arranged in an air monitor 12a, 12b which may be of the optical or acoustic type. Furthermore a pressure gauge 10a is connected to the drip chamber 10 for monitoring purposes.

The plasma conveyed through the line 8 from the plasma filter 6 is pumped with the help of a plasma pump 13 up to a plasma reservoir 14. With the help of a second pump 15 a citrate solution from a reservoir 17 for such a solution is conducted by a second pump 15 to the same reservoir through a line 16. This solution too is intended to prevent coagulation. Before any plasma is returned to the patient, this citrate solution is neutralized with the help of a calcium solution from a source 18 which is in connection with the drip chamber 10 through a line 19.

Plasma from the plasma reservoir 14 is pumped via a plasma pump 20, via one or the other of two valves 21 and 22. It is to be treated in one or the other of two columns 23 and 24 whilst the second of these columns is appropriately regenerated at the same time, as will be described in more detail in the following.

Between the valves 21 and 22 and the columns 23 and 24 two air monitors are arranged for the protection of the columns 23 and 24. These air monitors have been given the designations 25a, 25b and 26a, 26b respectively. The air monitors enclose transparent drip chambers 27 and 28 respectively. Also connected to these drip chambers are pressure gauges 29 and 30 respectively.

From the columns 23 and 24 the fluid treated is passed through simple protein detectors 31 and 32 respectively which may be adapted so that they transilluminate the line used directly if the same is made of a transparent material, e.g.

plastics. Alternatively a simple glass tube may be inserted in the line so as to facilitate the trans-illumination. The precision required is not great, however, so that the material used can be sufficiently inexpensive for the tube or the line to be discarded after use.

The protein detectors 31 and 32 are followed by two further valves 33 and 34 respectively. With the help of these valves the treated fluid is either returned to the patient via a line 35 or to a drain 36 or to a collecting receiver for a required separated fraction.

The division may take place, for example, in such a manner that the protein detectors 31 and 32 perceive when the treated fluid contains more than a certain percentage of protein, e.g. 70%. If this is the case, the fluid is conveyed through the valves 33 and 34 respectively to a pH meter 38. If the pH measured is too low, e.g. below 6, the fluid is passed via the valve 39 to the drain 36. If, on the other hand, the pH is suitable, the fluid is taken via a reservoir 40, a particle filter 41, a pump 42 and a heating device 43 to the drip chamber 10. This occurs thus through the line 35. To the line 35 may also be conducted a desired diluting and/or replacement liquid via a line 44 from a source 45 for such a liquid. This liquid may be constituted for example, substantially, of a sodium chloride solution. Should it be found however instead, with the help of the protein detectors 31 or 32, that the treated fluid contains less than the said percentage of protein, the fluid is passed via a pH meter 46 and a more sensitive protein contents meter 47 to a valve 48. If it is found with the help of these meters that the protein content and the pH are within certain specified limits, the treated fluid is conducted directly to the collecting receiver 37 for the separated fraction. If, on the other hand, the pH and protein content measured are outside the specified limits, the fluid is passed instead with the help of the valve 48 to the drain 36.

To allow the columns 23 and 24 to be flushed clean, it is possible with the help of the plasma pump 20 to feed a flushing liquid from a source 49 for such a liquid via a line 50.

Whilst one of the columns 23 and 24 is used for adsorption, the other one is appropriately regenerated with the help of an appropriate mixture of a base and an acid which with the help of pumps 51 and 52 are pumped from sources 53 and 54 respectively for such materials.

## Example

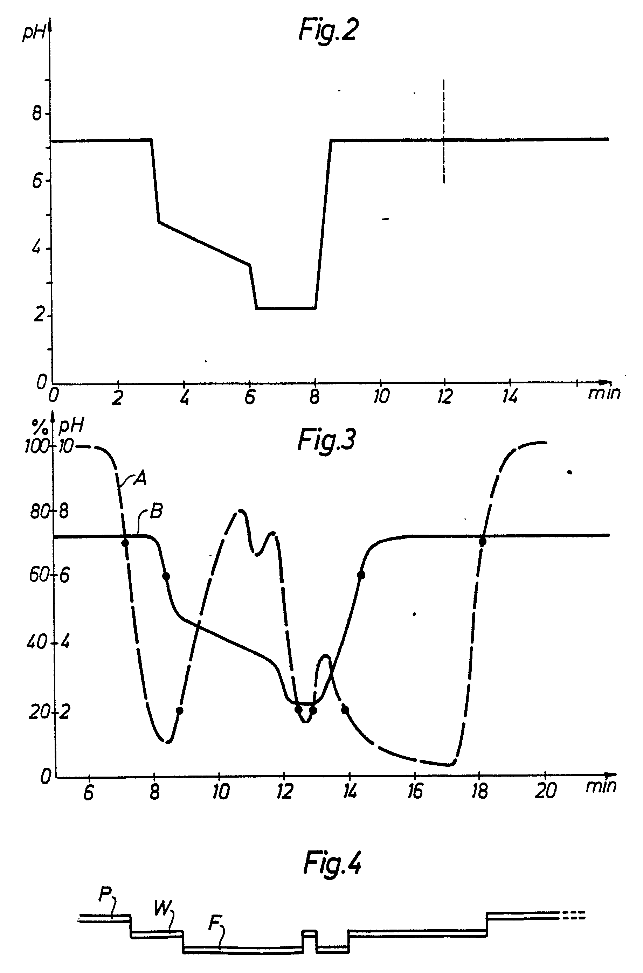

In the diagrams in fig. 2, 3 and 4 a typical regenerating profile for pH and protein values obtained is shown by way of example. In the procedure of the example two columns are used containing 50 ml of gel consisting of protein A attached to agarose of the type which is sold under the name of Sepharose CL4 B by Pharmacia Chemicals, Uppsala. The IgG concentration in the plasma was some 10 mg/ml. The flow of the eluent was approx. 15 ml/min.

In fig. 2 can be seen how the pH value varied at the input side of the columns 23 and 24. At time 0 the flushing of a saturated column was commenced. This flushing took place according to the example at pH value 7.2. After approx. 3 minutes of flushing the pH value dropped rapidly down to approx. 4.8 which is the approximate value at which the attached fraction commences to detach itself. In order to treat the column as well as the separated fraction with suitable care, the pH value is controlled slowly down to 3.5. Thereafter the value is lowered rapidly to approx. 2.2. At this value the final residues of the attached fraction are separated. After the separation the column is flushed, restoring the pH value to the value of 7.2. After this the column is ready for new adsorption.

In fig. 3 is shown what happens at the output side of the column when the pH value is controlled in accordance with fig. 2. Note the time shift on the time axis. The reaction on the output side of the column is thus taking place with a certain delay in relation to the control on the input side of the column. During the first flushing the protein content diminishes rapidly according to curve A and, as indicated by fig. 4, the plasma return P to the patient is interrupted at a plasma content of approx. 70%. When the plasma content falls below this value, the treated fluid W commences instead to be conveyed to the drain 36. As the pH value commences to drop in accordance with curve B, further protein A appears in the form of peaks on the curve A. With the help of the valve 48 the fluid F emerging from the regenerated column is then switched over from the drain 36 to the collecting receiver 37 in accordance with what is evident in fig. 4. Between the peaks of the curve A the protein content drops below the value of 20%. Such a diluted fraction is considered not to be worth collecting in accordance with the example and the valve 48 is made therefore to convey the liquid for a short while to the drain 36. Subsequently, however, a new protein top appears on curve A which is conducted to the collecting receiver 37. The value then drops again to below 20% so that connection to the drain 36 is established and is maintained until the protein content once more rises up to a value of over 70%, that is to say a while after a new plasma treatment has been started.

Naturally the invention is not limited exclusively to the example described above with its numerical data or to the preferred embodiment described above.

## Claims

1. An arrangement for the removal of one or more fractions out of whole blood, plasma or other similar body fluids with the help of adsorption on an adsorption column (23, 24) comprising means (20, 21, 22) for feeding said body fluid and a regenerating fluid (53, 54) in turn to the adsorption column, and means for the removal of said fluids from the adsorption column, the means for the removal of the fluids comprising a valve means (33, 34) which is adapted to control the flow from the column in dependence of an analysis carried out by a first analysing device (31, 32) to feed said flow either to a second analysing device (46, 47) for a second analysis of the fluid or to the source (1) of the body fluid e.g. a patient, the fluid analysed by the second analysing device being fed either to a drain (36) or to a collecting receiver (37), characterized in that the first analysing device (31, 32) is for a coarser analysis of the fluids than the second analysing device (46, 47).

2. An arrangement in accordance with claim 1, characterized by a third analysing device (38) for checking the fluid which is returned to the source (1) in combination with further valve means (39), the fluid analysed by the third analysing device being fed by means of said valve means (39) either to said source (1) or to the drain (36).

3. An arrangement in accordance with claim 1, characterized in that the said first analysing device (31, 32) is constituted by a detector for the monitoring of the degree of dilution of the treated fluid, e.g. a whole blood detector in the treatment of blood or a plasma detector in the treatment of plasma.

4. An arrangement in accordance with anyone of the preceding claims, characterized by a valve device (21, 22) arranged upstream of the column which is adapted so that it can convey alternately the treated fluid and regenerating liquid and/or a flushing liquid to the column.

5. An arrangement in accordance with claim 4, wherein the said regenerating liquid is adapted to lower the pH of the column gradually during a certain period, characterized in that means (51, 52) are adapted so that at the start of the period a rapid lowering is achieved so as to accelerate the starting of the regeneration, whereupon the lowering is adapted so as to occur slowly during the actual regeneration, and preferably to be terminated rapidly during the finish of the said period.

6. An arrangement in accordance with claim 1, characterized in that the second analysing device is constituted by a pH meter (46).

7. An arrangement in accordance with claim 2, characterized in that the third analysing device is constituted by a pH-meter (38).

8. An arrangement in accordance with claim 1, characterized in that the second analysing device is constituted by a protein detector (47) for the monitoring of the separated fraction.

9. An arrangement in accordance with any one of claims 6 and 8, characterized by a further valve device (48) arranged downstream of the second analysing device (46, 47) and controlled by the same for the conveying of the separated fraction to the collecting receiver (37).

10. An arrangement in accordance with claim 9, characterized in that the said further valve device (48) is adapted so as to be controlled by two analysing devices (46, 47) arranged upstream, namely a pH meter (46) and a protein detector (47).

11. An arrangement in accordance with any one of the preceding claims, characterized in that at least two adsorption columns (23, 24) are connected in parallel for alternate adsorption and regeneration.

12. An arrangement in accordance with any one of the preceding claims, characterized in that the adsorption column (23, 24) contains factor IX bound to agarose for the removal of antibodies against factor IX.

13. An arrangement in accordance with any one of claims 1—11 characterized in that the adsorption column (23, 24) contains protein A for the removal of one or several protein fractions of the IgG type.

**Patentansprüche**

1. Anordnung zum Entfernen einer oder mehrerer Fraktionen aus Vollblut, Plasma oder anderen, ähnlichen Körperfluids mit Hilfe von Adsorption an einer Adsorptionskolonne (23, 24), welche Mittel (20, 21, 22) für das Zuführen des Körperfluids und eines Regenerierungsfluids (53, 54) nacheinander zu der Adsorptionskolonne und Mittel zum Entfernen dieser Fluids aus der Adsorptionskolonne aufweist, wobei die Mittel zum Entfernen der Fluids ein Ventilmittel (33, 34) aufweisen, welches so ausgelegt ist, daß es den Fließstrom aus der Kolonne in Abhängigkeit von einer Analyse steuert, welche durch eine erste Analysevorrichtung (31, 32) durchgeführt wird, um den Fließstrom entweder zu einer zweiten Analysevorrichtung (46, 47) für eine zweite Analyse des Fluids oder zu der Quelle (1) des Körperfluids, z.B. einem Patienten, zu führen, wobei das von der zweiten Analysevorrichtung analysierte Fluid entweder zu einem Ablauf (36) oder zu einer Sammelaufnahme (37) zugeführt wird, dadurch gekennzeichnet, daß die erste Analysevorrichtung (31, 32) für eine grobere Analyse der Fluids ist als die zweite Analysevorrichtung (46, 47).

2. Anordnung nach Anspruch 1, gekennzeichnet durch eine dritte Analysevorrichtung (38) zum Prüfen des Fluids, welches zu der Quelle (1) zurückgeführt ist, in Kombination mit weiteren Ventilmitteln (39), wobei das von der dritten Analysiervorrichtung analysierte Fluid mittels der Ventilmittel (39) entweder zu der Quelle (1) oder zu dem Ablauf (36) geführt wird.

3. Anordnung nach Anspruch 1, dadurch gekennzeichnet, daß die erste Analysevorrichtung (31, 32) aus einem Detektor zum Überwachen des Verdünnungsgrades des behandelten Fluids aufgebaut ist, z.B. einem Vollblutdetektor bei der Behandlung von Blut oder einem Plasmadetektor bei der Behandlung von Plasma.

4. Anordnung nach einem der vorstehenden Ansprüche, gekennzeichnet durch eine Ventilvorrichtung (21, 22), welche stromaufwärts von der Kolonne angeordnet und so ausgelegt ist, daß sie wahlweise die behandelte Flüssigkeit und Regenerierungsflüssigkeit und/oder eine Spülflüssigkeit zu der Kolonne hinleiten kann.

5. Anordnung nach Anspruch 4, wobei die Regenerationsflüssigkeit so angepaßt ist, daß sie den pH der Kolonne nach und nach während eines bestimmten Zeitabschnittes verringert, dadurch gekennzeichnet, daß Mittel (51, 52) so angepaßt sind, daß zu Beginn des Zeitabschnittes ein schneller Abfall erreicht wird, um den Beginn der Regenerierung zu beschleunigen, woraufhin das Erniedrigen so angepaßt wird, daß es während der eigentlichen Regenerierung langsam vonstatten geht und vorzugsweise während des Endabschnittes des Zeitabschnittes schnell beendet wird.

6. Anordnung nach Anspruch 1, dadurch gekennzeichnet, daß die zweite Analysevorrichtung aus einem pH-Meter (46) aufgebaut ist.

7. Anordnung nach Anspruch 2, dadurch gekennzeichnet, daß die dritte Analysevorrichtung aus einem pH-Meter (38) aufgebaut ist.

8. Anordnung nach Anspruch 1, dadurch gekennzeichnet, daß die zweite Analysevorrichtung aus einem Proteindetektor (47) zur Überwachung der abgetrennten Fraktion aufgebaut ist.

9. Anordnung nach einem der Ansprüche 6 und 8, gekennzeichnet durch eine weitere Ventilvorrichtung (48), welche stromabwärts von der zweiten Analysevorrichtung (46, 47) angeordnet ist und von derselben für die Zuleitung der abgetrennten Fraktion zu der Sammelaufnahme (37) gesteuert wird.

10. Anordnung nach Anspruch 9, dadurch gekennzeichnet, daß die weitere Ventilvorrichtung (48) so ausgelegt ist, daß sie von den zwei Analysevorrichtungen (46, 47), welche stromaufwärts angeordnet sind, nämlich einem pH-Meter (46) und einem Proteindetektor (47), gesteuert wird.

11. Anordnung nach einem der vorstehenden Ansprüche, dadurch gekennzeichnet, daß mindestens zwei Adsorptionskolonnen (23, 24) für abwechselnde Adsorption und Regeneration parallel angeschlossen sind.

12. Anordnung nach einem der vorstehenden Ansprüche, dadurch gekennzeichnet, daß die Adsorptionskolonne (23, 24) für das Entfernen von Antikörpern gegen Faktor IX an Agarose gebundenen Faktor IX enthält.

13. Anordnung nach einem der Ansprüche 1 bis 11, dadurch gekennzeichnet, daß die Adsorptionskolonne (23, 24) für das Entfernen einer oder einiger Proteinfraktionen vom IgG-Typ Protein A enthält.

**Revendications**

1. Agencement pour l'extraction d'une ou plusieurs fractions du sang entier, du plasma ou d'autres fluides corporels similaires, par adsorption sur une colonne d'adsorption (23, 24), comprenant des moyens (20, 21, 22) pour amener successivement à la colonne d'adsorption ledit fluide corporel et un fluide de régénération (53, 54) et des moyens pour l'extraction de ces fluides hors de la colonne d'adsorption, les moyens d'extraction des fluides comprenant un système de vanne (33, 34), agencé pour commander l'écoulement hors de la colonne à la suite d'une analyse effectuée par un premier dispositif d'analyse (31, 32) afin d'acheminer ledit fluide soit vers un deuxième dispositif d'analyse (46, 47) pour une deuxième analyse du fluide, soit vers la source (1) du fluide corporel, par exemple un patient, le fluide analysé par le deuxième dispositif d'analyse étant acheminé vers un rejet (36) ou vers un récipient de collecte (37), caractérisé en ce que le premier dispositif d'analyse (31, 32) sert à une analyse des fluides plus grossière que le deuxième dispositif d'analyse (46, 47).

2. Agencement suivant la revendication 1, caractérisé par un troisième dispositif d'analyse (38) pour contrôler le fluide qui est restitué à la source (1), en combinaison avec une autre vanne (39), le fluide analysé par le troisième dispositif d'analyse étant dirigé par ladite vanne (39) soit vers ladite source (1), soit vers le rejet (36).

3. Agencement suivant la revendication 1, caractérisé en ce que le premier dispositif d'analyse (31, 32) est constitué par un détecteur de surveillance du degré de dilution du fluide traité, par exemple un détecteur de sang entier dans le traitement du sang ou un détecteur de plasma dans le traitement du plasma.

4. Agencement suivant l'une quelconque des revendications précédentes, caractérisé en ce qu'un système de vannes (21, 22) est prévu à l'amont de la colonne et est conçu de sorte qu'il peut diriger alternativement le fluide traité et un fluide de régénération et/ou un liquide de balayage vers la colonne.

5. Agencement suivant la revendication 4, dans lequel ledit liquide de régénération est prévu pour abaisser le pH de la colonne progressivement pendant une certaine période, caractérisé en ce que des moyens (51, 52) sont prévus de sorte que, au début de la période, on obtient une diminution rapide afin d'accélérer le démarrage de la régénération, après quoi la diminution est réglée de manière à se produire lentement pendant la régénération effective et à se terminer de préférence rapidement pendant l'achèvement de ladite période.

6. Agencement suivant la revendication 1, caractérisé en ce que le deuxième dispositif d'analyse est constitué par un pH-mètre (38, 46).

7. Agencement suivant la revendication 2, caractérisé en ce que le troisième dispositif d'analyse est constitué par un pH-mètre (38).

8. Agencement suivant la revendication 1, caractérisé en ce que le deuxième dispositif d'analyse est constitué par un détecteur de protéine (47) pour le contrôle de la fraction séparée.

9. Agencement suivant l'une quelconque des revendications 6 et 8, caractérisé en ce qu'une autre vanne (48) est prévue à l'aval du deuxième dispositif d'analyse (46, 47) et est commandée par celui-ci pour diriger la fraction séparée vers le récipient de collecte (37).

10. Agencement suivant la revendication 9, caractérisé en ce que ladite autre vanne (48) est prévue pour être commandée par deux dispositifs d'analyse (46 et 47) placés à l'amont, plus particulièrement un pH-mètre (46) et un détecteur de protéine (47).

11. Agencement suivant l'une quelconque des revendications précédentes, caractérisé en ce qu'au moins deux colonnes d'adsorption (23, 24) sont reliées en parallèle pour adsorption et régénération alternées.

12. Agencement suivant l'une quelconque des revendications précédentes, caractérisé en ce que la colonne d'adsorption (23, 24) contient du facteur IX fixé à de l'agarose, pour l'élimination des anticorps contre le facteur IX.

13. Agencement suivant l'une quelconque des revendications 1 à 11, caractérisé en ce que la colonne d'adsorption (23, 24) contient de la protéine A pour l'extraction d'une ou plusieurs fractions de protéine du type IgG.

## Fig.1

## Fig.2

## Fig.3

## Fig.4